Europäisches Patentamt

European Patent Office

Office européen des brevets

(11) Numéro de publication: **0 435 851 A1**

## (12) DEMANDE DE BREVET EUROPEEN

(21) Numéro de dépôt: **91101049.4**

(51) Int. Cl.5: **G01N 33/543**

(22) Date de dépôt: **24.09.87**

Cette demande a été déposée le 28 - 01 - 1991 comme demande divisionnaire de la demande mentionnée sous le code INID 60.

(30) Priorité: **30.09.86 FR 8613782**

(43) Date de publication de la demande:
**03.07.91 Bulletin 91/27**

(60) Numéro de publication de la demande initiale en application de l'article 76 CBE : **0 266 278**

(84) Etats contractants désignés:
**BE CH DE ES FR GB GR IT LI LU NL SE**

(71) Demandeur: **INDICIA Société Civile d'Etudes et de Recherches (S.C.E.R.)**
**87 rue de la République**
**F-69600 Oullins(FR)**

(72) Inventeur: **Serres, Pierre-François**
**33, avenue de la Californie**
**F-69600 Oullins(FR)**

(74) Mandataire: **Ropital-Bonvarlet, Claude**
**Cabinet BEAU DE LOMENIE, 51, avenue Jean-Jaurès**
**F-69007 Lyon(FR)**

(54) **Procédé de dosage de substances d'intérêt clinique réactives immunologiquement.**

(57)
- Procédé de dosage.
- L'invention concerne un procédé de dosage d'une substance d'intérêt clinique réactive immunologiquement par d'autres substances du même type (dosage d'un antigène par un anticorps et réciproquement), du type selon lequel :
  . on greffe préalablement une substance active immunologiquement, jouant le rôle de réactif doseur sur des microparticules naturelles ou synthétiques,
  . on agglutine lesdites microparticules sur la substance à doser d'intérêt clinique réactive immunologiquement,
  . on mesure, par lecture, le résultat issu de la réaction d'agglutination que l'on compare à une gamme étalon, caractérisé en ce que la phase d'agglutination s'effectue en soumettant lesdites microparticules à un mouvement périodique de fréquence comprise entre 4 et 40 Hertz et d'amplitude variant de quelques millimètres à quelques centimètres.
- Application au dosage auantitatif d'anticorps et d'antigène.

EP 0 435 851 A1

EP 0 435 851 A1

## PROCEDE DE DOSAGE DE SUBSTANCES D'INTERET CLINIQUE REACTIVES IMMUNOLOGIQUEMENT

La présente invention concerne un procédé de dosage de substances d'intérêt clinique réactives immunologiquement. Elle concerne également un dispositif pour la mise en oeuvre de ce procédé.

Par substances réactives immunologiquement, on entend principalement toutes les antigènes (incluant les haptènes), et les anticorps (monoclonaux ou polyclonaux) obtenus par fusion cellulaire ou immunisation naturelle ou provoquée.

Par substances d'intérêt clinique réactives immunologiquement, on entend principalement toutes les molécules antigéniques ou anticorps dont le dosage, dans un échantillon biologique d'origine humaine ou animale, peut présenter un intérêt dans le diagnostic médical, la recherche clinique ou le suivi d'un processus pathologique ou d'une thérapeutique mise en oeuvre.

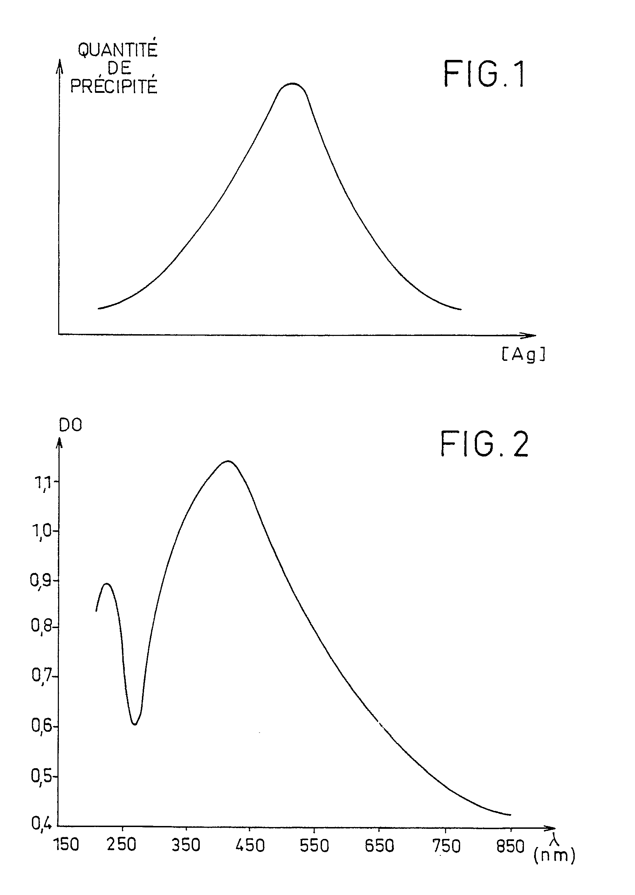

Le phénomène immunologique exploité dans ces procédés de dosage immunologique est représenté dans la **fig. 1**.

Les procédés de dosage les plus couramment utilisés, basés sur l'exploitation de la courbe de la **fig. 1** sont :
- l'immuno-diffusion radiale,
- la radio-immunologie, l'immuno-enzymologie, l'immuno-fluorescence, la néphélémétrie,
- d'autre part, depuis quelques années, quelques essais de versions quantitatives du "latex immuno essai", à savoir l'agglutination de microsphères synthétiques, ont été décrits.

Jusque là, ces techniques largement employées et depuis longtemps (**SINGER** J.M. and **PLOTZ** C.M. the Latex fixation Test, American Journal of Medicine 21, 888 (1956), à cause de leur simplicité et de leur faible coût ne présentaient qu'un aspect qualitatif (lame) ou semi-quantitatif (cupule). Les formes qualitatives et semi-quantitatives (lame-carte-cupule) du latex immuno-essai restent d'ailleurs, à l'heure actuelle, très employées dans le diagnostic clinique, et font l'objet de nombreux brevets et publications, en particulier dans le domaine de la virologie et microbiologie (**KHABBAZ** R.F., H.C. **STANDFORD** and Coll. 1985, Measurement of amikacin in serum by a Latex agglutination inhibition test, Journal of Clinical Microbiology 22 : 699-701, brevet **US-3 488 156**, demande de brevet **EP 186 946**).

Dans ces techniques de tri ou de dépistage, l'antigène ou l'anticorps est fixé sur des microsphères synthétiques et l'absence ou la présence d'agglutination, par l'antigène ou l'anticorps correspondant, est appréciée en tube ou en cupule, de manière visuelle ou turbidimétrique.

Les quelques essais de latex immuno-essai quantitatifs proposés dans la littérature (par exemple **RIPOLL** J.P. **ROCH** A.M. **QUASH** G.A. and J. **GRANGE**, 1980, Journal of Immunological Methods 33, 159-173 demandes de brevet **EP 189 389** et 5978) présentent essentiellement trois phases :

### Phase I :

Phase de fixation de l'antigène ou de l'anticorps sur une microsphère de polymère synthétique, de taille comprise entre 0,01 micromètre et 5 micromètres. La fixation est réalisée par tous moyens connus, identiques à ceux décrits dans les techniques sur lame (par exemple : brevet américain **US-A-4 217 338**).

### Phase II :

Phase d'agglutination immunologique des microsphères synthétiques porteuses de l'antigène ou de l'anticorps.

Cette phase II du procédé, à savoir l'agglutination immunologique des microsphères synthétiques, doit tendre vers deux buts :
- la réduction maximum de l'agglutination non spécifique des particules ; par agglutination spécifique on entend l'agglutination des particules due à des interactions faibles (du type liaison hydrogène, ionique, ou Van der Waals), entre les microsphères ou les protéines fixées sur celles-ci.
- l'augmentation maximum de l'agglutination spécifique (immunologique) des particules.

Au cours de cette phase, des moyens essentiellement chimiques ou biochimiques sont mis en oeuvre pour stabiliser les particules, afin de minimiser l'agglutination non spécifique.

Pendant cette phase, des moyens sont également mis en oeuvre, afin d'augmenter l'agglutination spécifique des particules et donc la précision du dosage.

Pour réduire l'agglutination non spécifique des particules, le brevet américain **US-A-4 329 152** propose de les stabiliser par fixation d'albumine bovine rendue électro-négative à un pH sensiblement égal à 10. Le

2

pH très basique imposé de la sorte au réactif et aux gammes étalon est incompatible avec la survie des protéines et en fait un réactif difficilement utilisable. D'autre part, la fiabilité du procédé en est réduite, car les solutions tampons à des pH si basiques dont instables. D'autre part, l'albumine bovine fixée par liaison hydrophobe se détache progressivement des particules à pH 10 et perd progressivement sa capacité de stabilisation.

Les agents chaotropiques utilisés quelquefois pour réduire l'agglutination non spécifique des particules présentent malheureusement l'inconvénient d'affaiblir ou de rompre les liaisons antigènes-anticorps.

D'autres procédés de stabilisation très compliqués et inutilisables dans un réactif industriel mettent en jeu des mélanges de particules légères et lourdes (par exemple demande de brevet **EP 163 321**) et des centrifugations.

Pour favoriser l'agglutination spécifique des particules, c'est-à-dire l'agglutination immunologique, les procédés décrits utilisent une agitation traditionnelle, thermostatée dans un bain marie à 37° C ou 40° C, pendant des durées variant de 1/2 heure à une heure. De telles durées d'incubation à 37° C, outre qu'elles favorisent l'agglutination non spécifique indésirable des particules, imposent un chronométrage rigoureux et fastidieux dans l'introduction initiale du réactif dans chaque tube de la série et dans le processus d'arrêt de la réaction de la série de tubes.

L'augmentation de l'agglutination spécifique (immunologique des particules) est quelquefois pratiquée en ajoutant, dans le milieu réactionnel, des additifs comme par exemple, de la dextrose connue sous la marque commerciale "Dextran" ou du polyéthylène glycol. L'inconvénient de ces substances est qu'elles sont souvent largement hydrophobes et qu'elles se fixent donc sur les fragments Fc des anticorps fixés sur les microparticules, fragments eux-mêmes très hydrophobes, entraînant donc une agglutination non immunologique des particules et induisant ainsi des interférences et par la même des faux positifs aux réactions.

C'est l'un des buts de la présente invention de supprimer l'agglutination non spécifique des particules et d'augmenter simultanément l'agglutination immunologique spécifique des particules, sans présenter tous les inconvénients décrits ci-dessus, et de surcroît, en diminuant considérablement la durée de l'opération.

**Phase III** :

Phase de lecture du résultat.

Actuellement, les techniques de lecture du résultat de l'agglutination dans les latex-immuno-essais quantitatifs sont :
- le comptage de particules,
- l'opacimétrie, encore appelée turbidimétrie (pratiquée quelquefois dans le visible ou l'infra-rouge, le plus souvent dans le proche infra-rouge),
- l'analyse centrifuge.

La néphélémétrie laser mesure la lumière diffusée par les agrégats de particules de latex. Cette technique, décrite par exemple dans l'article suivant : **GRANGE J., ROCH A.M.**, and **G.A. QUASH**, 1977, Journal of Immunogical Methods, 18, 326-375, présente l'inconvénient de nécessiter un appareillage de lecture coûteux et sophistiqué.

Le comptage de particules, décrit par exemple dans l'article suivant : **MAGNUSSON C.G.**, **MASSON P.L.**, Journal of Allergy Clin, and Immunol., 70:326, 1982, nécessite également un appareillage complexe et coûteux, à la portée de peu de laboratoires.

L'opacimétrie dans le visible, également dénommée turbidimétrie dans le visible, mesure la lumière transmise par la suspension de particules, c'est-à-dire la lumière qui n'est ni absorbée par les particules, ni diffusée par celles-ci. L'opacimétrie ou turbidimétrie des latex immuno-essais, qualitatifs ou quantitatifs, doit être pratiquée à une longueur d'onde assez proche de la taille des particules. La validité de la mesure turbidimétrique impose des suspensions très diluées, afin de pouvoir visualiser l'effet écran souhaité et, comme il est indiqué dans le Certificat d'Addition n° **78-28 250** du brevet français n° **77-25 049**, afin de minimiser l'absorption de lumière par les particules.

Les mesures turbidimétriques (ou opacimétriques) dans le visible des latex immuno-essais décrites dans la littérature (exemple : **BERNARD A.M., LAUWERYS R.R.**, 1982 Clinica Chemica Acta 119, 335-339) utilisent, selon la taille de la particule, différentes longueurs d'ondes du visible, par exemple 360, 400, 450 et beaucoup plus généralement, entre 600 et 750 nanomètres. La précision des résultats turbidimétriques est médiocre, car les suspensions de particules doivent être très diluées, afin, comme on l'a dit, d'obtenir les variations d'effet-écran souhaitées et afin de minimiser l'absorption des latex. D'ailleurs, il faut généralement dans ces techniques, pour augmenter la précision du résultat, utiliser des cuves de lecture dont le chemin optique est important, de l'ordre de deux à quatre centimètres.

Afin d'utiliser des suspensions de latex plus concentrées et d'augmenter ainsi des dosages et afin de ne pas être gêné par l'absorption des latex dans le visible ou dans l'ultraviolet, le brevet français **FR-A-77 25 049** et son Certificat d'Addition **FR-A-78 28 250**, préconisent, pour la lecture des particules, l'opacimétrie infra-rouge. En effet, les latex n'absorbent plus la lumière dans l'infra-rouge. L'inconvénient de cette technique est d'imposer un appareillage coûteux et sophistiqué pour la lecture opacimétrique infra-rouge.

Le but essentiel de la présente invention est donc de remédier aux nombreux inconvénients (des phases II et III soulignés précédemment, par la mise en oeuvre d'un procédé et d'un appareillage d'agglutination simple et nouveau, faisant ainsi de ce type de dosage un procédé très simple de mise en oeuvre, très rapide et à la portée de n'importe quel laboratoire.

Le procédé selon l'invention de dosage de substances d'intérêt clinique réactives immunologiquement est donc du type dans lequel :
- on greffe préalablement une substance active immunologiquement, jouant le rôle de réactif doseur, sur des microparticules,
- on agglutine lesdites microparticules par la substance d'intérêt clinique réactive immunologiquement,
- on mesure par lecture le résultat issu de la réaction d'agglutination que l'on compare à une gamme étalon.

Le procédé de dosage selon l'invention se caractérise en ce que la phase d'agglutination s'effectue en soumettant lesdites microparticules greffées à un mouvement périodique de fréquence comprise entre 4 et 40 Hertz, et de préférence 20 hertz, et d'amplitude variant de quelques millimètres à quelques centimètres, de préférence de l'ordre de 4 millimètres.

En effet, on a constaté que si la fréquence était inférieure à 4 Hertz, cela diminuait l'intérêt de la méthode du fait que l'on augmente la durée de la phase d'agglutination, afin d'obtenir un résultat utilisable et du fait que l'effet recherché sur l'agglutination non spécifique devient médiocre ; par contre, si la fréquence est supérieure à 40 Hertz, on provoque la rupture des agrégats (Immuns complexes) formés par les microparticules agglutinées.

Comme il apparaît dans la description qui précède, dans la phase d'agglutination (phase II du procédé), afin de remédier à tous les inconvénients des procédés chimiques décrits ci-dessus visant à diminuer l'agglutination non spécifique des particules, ou augmenter l'agglutination immunologique de celles-ci, le moyen d'agglutination mis en oeuvre selon l'invention utilise un procédé mécanique qui atteint simultanément ces deux buts.

Ce procédé mécanique est basé sur l'observation que l'agglutination non spécifique, qui est liée à des interactions faibles entre les particules (liaisons de type ionique, hydrogène, Van der Waals), est rendue impossible ou est fortement inhibée par un mouvement périodique imprimé à celles-ci, de très faible amplitude et de très grande fréquence, quelle que soit la trajectoire du mouvement périodique (par exemple et commodément rectiligne, circulaire ou ovalisée). On a également montré qu'un tel procédé favorise notablement l'agglutination spécifique (immunologique) des microparticules porteuses de l'antigène ou de l'anticorps correspondant et donc permet une réaction spécifique rapide et effectuée, de surcroît, à température ambiante.

Avantageusement, en pratique :
- la trajectoire d'agglutination s'effectue à température ambiante,
- la durée du mouvement périodique imposée aux microparticules, durant la phase d'agglutination, est fonction de la fréquence de la vibration, de la trajectoire et de l'amplitude du mouvement. Elle est avantageusement de 10 minutes pour une fréquence de 20 Hertz et un mouvement circulaire périodique de 4 millimètres de diamètre,
- les microparticules, aptes à la lecture spectrophotométrique d'absorption ultra-violet-visible, sont constituées de matériaux naturels ou synthétiques, de toute nature absorbante, notamment de polystyrène, polyvinyl-toluène, acrylonitrile, styrène-butadiène, copolymères acrylonitrile - acide acrylique, ester acrylique, poly-vinylbutadiène, et de préférence de polystyrène,
- les microparticules sont de forme quelconque mais de préférence sous forme de microsphères,
- le diamètre des microsphères naturelles ou synthétiques est compris entre 0,01 et 5 micromètres, et de préférence voisin de 0,8 micromètre,
- les microparticules sont constituées d'un matériau absorbant mais non coloré,
- il est possible d'utiliser des microparticules absorbantes qui sont colorées ; dans ce cas, la lecture du résultat par spectrophotométrie d'absorption ultra-violet-visible est effectuée, soit à longueur d'onde correspondant à l'absorption maximum de la matière de la particule non agglutinée ($\lambda$ max), soit à la longueur d'onde correspondant à l'absorption maximum de la couleur de la particule considérée.

L'invention concerne également un dispositif pour la mise en oeuvre du procédé. Ce dispositif est caractérisé en ce qu'il comporte au moins :
- une première série de tubes renfermant au moins une gamme étalon lyophilisée de la substance à doser,

- une seconde série de tubes renfermant une substance active immunologiquement et jouant le rôle de concentration en poids des microparticules étant comprise entre 0,1 et 10 pour cent,
- une troisième série de tubes renfermant un échantillon lyophilisé de la solution de dilution de la gamme étalon.

La manière dont l'invention peut être réalisée et les avantages qui en découlent ressortiront mieux de l'exemple de réalisation qui suit, donné à titre indicatif et non limitatif, à l'appui des figures annexées.

La **fig. 1** est un diagramme représentatif du phénomène immunologique exploité.

La **fig. 2** est un diagramme représentatif du spectre d'absorption ultra-violet-visible d'une suspension aqueuse de microsphères (non agglutinées) de polystyrène (non coloré) de 0,8 micromètre de diamètre.

La **fig. 3** est un diagramme représentatif d'un dosage d'Immuno-gammaglobulines G humaines par le procédé selon l'invention.

La **fig. 4** est une vue du boîtier compartimenté relatif au procédé de mise en oeuvre de l'invention.

L'exemple qui suit correspond au dosage d'antigènes au moyen d'anticorps greffés sur des microsphères. Il est bien entendu que l'on pourrait tout aussi bien procéder au dosage d'anticorps au moyen d'antigènes, en suivant le même processus mais en remplaçant les antigènes par les anticorps et réciproquement.

Avant le dosage proprement dit, on procède tout d'abord au greffage des anticorps sur des microsphères synthétiques calibrées, dénommées également latex. Ceux-ci sont réalisés par exemple en polystyrène, polyvinyl-toluène, acrylonitrile, styrène-butadiène, copolymère acrylonitrile-acide acrylique, ester acrylique, polyvinyl-butadiène, etc. Les particules de latex K 140, K 109, K 610, Ψ 512, Ψ 513, Ψ 480, Ψ 502 (-COOH) ou Ψ 169, Ψ 181 (-CONH2), vendus sous ces dénominations commerciales par **RHONE-POULENC**, conviennent par exemple très bien au procédé.

Le greffage des anticorps sur les microsphères synthétiques s'effectue de manière connue, c'est-à-dire par fixation ionique, hydrophobe ou covalente.

Lorsque l'on fait appel à des accrochages de type ionique ou hydrophobe d'anticorps sur des microsphères de latex, ces accrochages, qui se font de manière connue par simple contact de la protéine et du support, conduisent à des phénomènes de détachement (également dénommés relargages) des protéines du support. L'une des raisons des phénomènes de relargage est qu'il existe des échanges permanents entre la population d'anticorps du type immuno-gammaglobulines G ainsi fixée et la population desdites immuno-gammaglobulines G fréquemment présente dans l'échantillon à analyser.

Avantageusement, les méthodes de fixation covalente sont plus fiables et préférables dans ce type de procédé de dosage immunologique. Les méthodes de fixation covalente d'anticorps ou d'antigène sur des microsphères synthétiques, ou latex, sont bien connues, de sorte qu'il n'y a pas lieu de les décrire ici plus en détail.

Dans le procédé selon l'invention, lorsque l'on met en jeu une fixation du type covalente, on fait appel avantageusement à un procédé de fixation qui n'a jamais été décrit dans ce type d'application et qui fournit de bons résultats.

Selon ce procédé de couplage, les radicaux fonctionnels présents sur les microsphères et mobilisés sont principalement les suivants : - COOH, - CONH2, - CONH - NH2, - COOCH3.

Ces radicaux sont transformés en acyl-azotures (-CON3) par le procédé qui va être décrit, l'azoture réagissant avec des radicaux -NH2, -SH ou OH des molécules biologiques (antigènes ou anticorps) que l'on veut greffer.

Par exemple, les latex Ψ 169 et Ψ 181 sont traités tout d'abord à l'hydrazine en solution puis ensuite aux vapeurs de nitrite acide de sodium, dans des conditions douces, avant d'être mis en contact avec un mélange poly-L-lysine-anticorps. Les latex carboxylés Ψ 480 ou Ψ 502 sont carboxyméthylés au méthanol-acide, puis traités à l'hydrazine en solution, puis aux vapeurs de nitrite-acide de sodium dans des conditions douces, avant d'être greffés dans un rapport approprié avec un mélange poly-L-lysine anticorps.

**Exemple :**

Greffage d'anticorps anti-immuno-gammaglobulines G humaines et anti β 2 micro-globuline humaine sur microsphères de polystyrène Ψ 480 et Ψ 502 (**RHONE-POULENC**) de diamètre respectif 0,8 et 0,85 micromètre.

Ce greffage se déroule suivant différentes phases résumées ci-dessous :

- méthylation des groupements carboxylés de latex : l'estérification des carboxyles libres des latex s'effectue dans un bain de méthanol acidifié, trois jours minimum, dix jours maximum, le plateau de saturation se trouvant en général à une moyenne de cinq jours,

5

- rinçage aqueux énergique,
- traitement des latex carboxyméthylés à l'hydrazine pendant 1 à 15 heures avec agitation à 20°,
- rinçage aqueux énergique, à 0° C,
- traitement aux vapeurs de nitrite de sodium acidifié par de l'acide chlorhydrique (conditions douces) ; les constituants du mélange et le mélange étant réalisés extemporanément et la réaction étant effectuée à 0° C,
- rinçage énergique avec tampon de couplage,
- greffage du mélange anticorps-poly-L-lysine ; la poly-L-lysine ainsi couplée aux latex-anticorps a un effet de saturation des sites de greffage (-CON3) et stabilise stériquement le réactif lors de sa conservation ; les proportions anticorps-poly-L-lysine varient selon l'enrichissement désiré en anticorps sur la microsphère synthétique et selon que l'on désire utiliser la partie droite ou gauche par rapport au point d'équivalence de la courbe représentée au sein de la **fig. 1** ; il est à noter que le procédé selon l'invention permet l'utilisation aussi bien de la partie droite que de la partie gauche de ladite courbe.
- désorption des anticorps fixés par liaison ionique (KCl 3 M, pendant 15 minutes) et par liaison hydrophobe (KSCN 1M, pendant 10 minutes),
- rinçage,
- stockage.

Il va de soi que si les groupes fonctionnels des particules sont -CONH2 (exemple Ψ 169 et Ψ 181) ou -COOCH3, la phase à l'hydrazine représente la première phase du procédé de greffage.

La phase suivante correspond à la phase d'agglutination des microsphères, qui peut être effectuée de la façon suivante. On soumet les microsphères de latex à un mouvement circulaire périodique de fréquence égale à 20 Hertz et d'amplitude de 4 mm, et ce, pendant une durée de dix minutes. Cette phase s'effectue à température ambiante, d'où ue diminution notable de l'appareillage (pas de bain marie ou de tout autre type d'appareillage de chauffage). Cette phase d'agglutination, ainsi réalisée, permet une forte diminution de l'agglutination non spécifique des particules. En effet, son efficacité sur l'agglutination non spécifique des particules est très grande : l'agglutination non spécifique, lorsque l'on utilise l'un des procédés précédemment décrits, c'est-à-dire à 37° C, pendant 1/2 heure au bain marie avec une agitation traditionnelle, est de l'ordre de 30 à 40 pour cent. L'agglutination non spécifique, selon le procédé décrit dans l'invention, varie de 2 à 5 pour cent seulement.

De plus, ce procédé ne nécessite pas de stabilisation de particules par de l'albumine bovine ou tout autre agent ionique rendu électro-négatif ou électro-positif à des pH très basiques, ou très acides, incompatibles avec la conservation des réactifs.

Le procédé permet donc, par voie de conséquence, le maintien du réactif et des gammes étalon à des pH physiologiques (voisin de 7) assurant donc une très bonne intégrité et une excellente conservation des constituants. Il est à noter qu'il existe un autre avantage en évitant l'utilisation de substances stabilisatrices protéiques, comme le permet le procédé. En effet, les substances stabilisatrices protéiques, telles que l'albumine, sont source de contamination bactérienne, ce qui est très gênant dans un réactif industriel devant être conservé plusieurs mois.

De plus, le procédé ci-dessus permet simultanément, avec une diminution très nette de l'agglutination non spécifique des particules, une augmentation notable de l'agglutination spécifique immunologique des particules.

Un plateau de saturation dans l'agglutination immunologique des microsphères est atteint rapidement en dix minutes environ. Une durée d'incubation si courte, outre qu'elle soit très intéressante pour l'utilisateur qui a un résultat de dosage extrêmement rapide, n'implique, d'autre part, pas de chronométrage particulièrement fastidieux, dans l'introduction initiale du réactif ou dans la phase d'arrêt de la réaction. De plus, l'efficacité du procédé selon l'invention sur l'agglutination spécifique est telle que l'agitation peut être effectuée à température ambiante, ce qui évite, comme déjà dit précédemment, l'utilisation d'appareillages chauffants ou de bains-marie d'où un avantage certain et appréciable de commodité.

Selon l'invention, on a donc vérifié expérimentalement que l'agglutination non spécifique, qui est liée à des interactions faibles entre les particules (liaison de type ionique, hydrogène, Van der Waals) est rendue impossible ou fortement inhibée par un mouvement périodique imprimé à celle-ci, de très faible amplitude et de très grande fréquence, quelle que soit la trajectoire du mouvement périodique (par exemple et commodément rectiligne, circulaire ou ovalisée). On a également vérifié qu'un tel procédé favorise notablement l'agglutination spécifique (immunologique) des microsphères synthétiques porteuses de l'antigène ou de l'anticorps en présence de l'antigène ou de l'anticorps correspondant et donc permet une réaction spécifique rapide et effectuée à température ambiante.

La phase d'agglutination étant réalisée, on provoque une dilution aqueuse du milieu d'incubation contenant lesdites microsphères jusqu'à un volume de 1 à 4 millilitres. La dilution peut être effectuée avec de l'eau, une solution saline ou tout autre solution tampon convenable. Le volume de dilution correspond à celui d'une cuve traditionnelle de spectrophotométrie de dimension 1 × 1 cm.

A titre d'exemple, la **fig. 2** représente le maximum d'absorption dans la zone ultra-violet-visible d'une suspension concentrée de microsphères de polystyrène non coloré, d'un diamètre de 0,8 micromètre, le spectre étant identique lorsque les microsphères sont revêtues ou non d'un anticorps ou d'un antigène.

Il est à noter que ce procédé est utilisable quelle que soit la nature du polymère (notamment ceux précisés précedemment), quelle que soit la taille des particules et leur coloration.

Le dispositif de mise en oeuvre du procédé décrit précédemment comprend, outre un spectrophotomètre d'absorption de modèle courant, un boîtier compartimenté et un moyen apte à imposer un mouvement vibratoire à la solution contenant les microsphères.

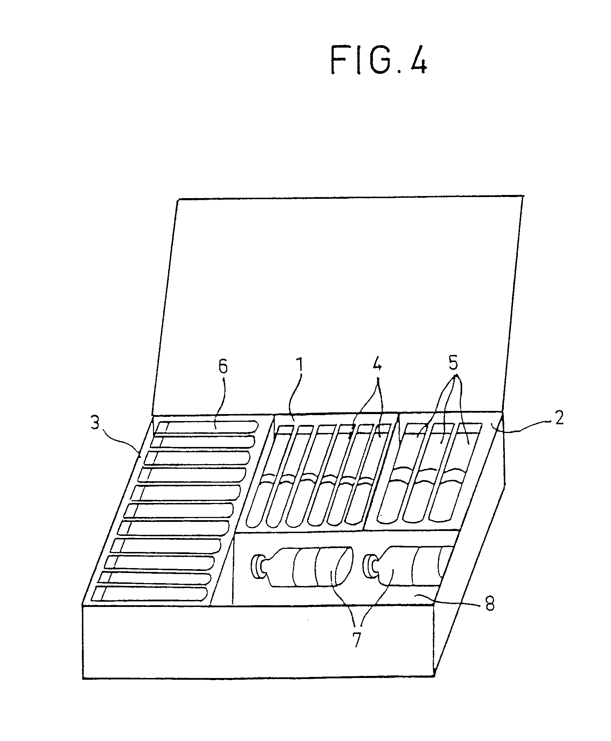

Le boîtier compartimenté, présenté en **fig. 4** en forme parallélépipède rectangle, a pour dimensions 13 × 16 × 4 cm et est réalisé en matière plastique ou en carton rigide. Il présente quatre compartiments :
- le premier compartiment **1** renferme une pluralité de tubes à hémolyse **4** horizontaux et parallèles, fermés et étiquetés (portant la concentration d'antigènes) et contenant 2 à 4 gammes étalon sous forme lyophilisée,
- le deuxième compartiment **2** renferme également une pluralité de tubes à hémolyse **5**, mais de taille plus importante, horizontaux et parallèles et contenant le réactif prêt à l'emploi, en l,occurrence les anticorps greffés sur des microsphères baignant dans un diluant approprié. En effet, le diluant doit assurer la conservation des anticorps, leur stabilité dans le temps et leur bonne intégrité. Ces tubes sont au nombre de quatre par gamme étalon et renferment 2,5 millilitres de réactif, la concentration en poids des microparticules dans le réactif est avantageusement comprise entre 0,1 et 10 pour cent et plus particulièrement entre 1 et 5 pour cent.
- le troisième compartiment **3** comporte une pluralité de tubes fermés **6** de petit diamètre, renfermant un échantillon de la solution de dilution de la gamme étalon sous forme lyophilisée. La quantité de solution de dilution est, de façon avantageuse, la quantité nécessaire à la dilution d'un échantillon biologique, par exemple la quantité nécessaire pour effectuer la dilution au dixième d'un échantillon biologique qui a déjà été dilué par de l'eau distillée. Une quantité de 0,45 ml ou 0,9 ml convient par exemple très bien.
- le quatrième compartiment **8** comporte au moins un flacon **7** de concentré du liquide de dilution.

Le moyen apte à assurer le mouvement périodique de haute fréquence et de faible amplitude des microsphères comprend avantageusement un moteur solidaire d' un axe vertical actionnant une came. Cette came communique à un plateau, lui-même solidaire d'un support de tubes parallèles, un mouvement périodique de haute fréquence et de faible amplitude et de trajectoire circulaire. Ledit plateau, de forme générale allongée, reste toujours parallèle à sa direction originale. Le moyen apte à assurer le mouvement périodique comprend, en outre, un châssis sur lequel on règle l'affichage de la vitesse de rotation du moteur et la mise en marche. L'amplitude du mouvement est limitée, dans le cas présent, à 4 mm et la fréquence affichée est de 20 Hertz.

Ainsi, lorsque l'on désire procéder à un dosage en antigène d'une solution, par exemple au dosage de la $\beta2$ micro-globuline dans un sérum d'insuffisant rénal, on prélève tout d'abord 50 microlitres du sérum à analyser, auquel on ajoute 0,45 ml d'eau distillée. De cette solution ainsi réalisée, on prélève 50 microlitres que l'on met dans des tubes **6** du compartiment **3** préalablement reconstitué avec 0,45 ml d'eau distillée. On procède à une homogénéisation classique. Puis, on prélève 50 microlitres de ce tube, on les mélange avec 50 microlitres du réactif prêt à l'emploi, c'est-à-dire de l'anticorps correspondant, greffés sur les microsphères et présents dans l'un des tubes **5** du compartiment **2** et qui a été préalablement homogénéisé. La concentration en poids des microsphères est avantageusement environ 1 pour cent. La nouvelle solution ainsi formée est placée sur le support solidaire du plateau déjà en mouvement. Elle subit ce mouvement périodique pendant une durée de dix minutes, la fréquence de vibration étant de 20 Hertz et l'amplitude de 4 mm. Cete phase d'agglutination étant réalisée, on procède à la phase de dilution. Le volume obtenu passe de 100 microlitres à 3,6 ml, c'est-à-dire au volume correspondant à la cellule d'analyse spectrophotométrique. On verse alors le contenu du tube dans la cellule de lecture de 1 × 1 cm et on procède à une analyse spectrophotométrique au maximum d'absorption dans l'ultra-violet-visible des microsphères non agglutinées, en l'occurence à 380 nanomètres, c'est-à-dire à la frontière du visible et de l'ultra-violet. On peut également effectuer la lecture en plaçant directement les tubes à hémolyse dans le spectrophotométre.

Au préalable, l'une des gammes étalon, présente dans le compartiment **1** du boîtier, a été utilisée afin de tracer la droite représentative de la relation entre la densité optique et le logarithme de la concentration en antigène. Une fois la densité optique de la solution obtenue déterminée, on la compare à la courbe étalon et l'on en déduit de façon extrêmement rigoureuse la concentration en antigènes du sérum initial.

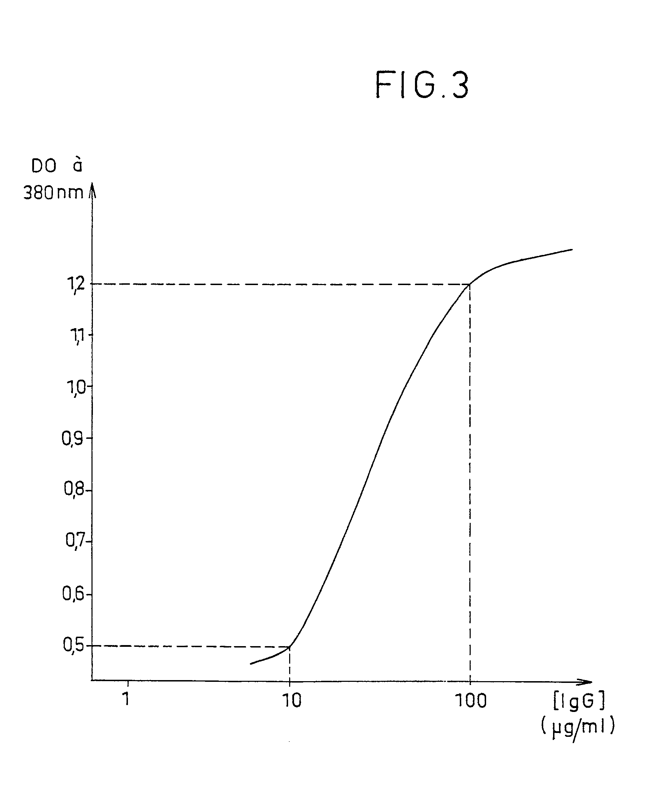

La **fig. 3** est une illustration d'un dosage d'Immuno-gamma-globulines G humaines au moyen du procédé et du dispositif conforme à l'invention. Dans cet exemple, c'est la partie droite par rapport au point d'équivalence de la courbe de la **fig. 1** qui est exploitée. Le coefficient de corrélation avec la néphélémétrie laser obtenu pour ce dosage sur cent sérums et liquides céphalorachidiens étant voisin de 1.

Le tableau I suivant est un tableau comparatif entre le procédé selon l'invention et un radio-immuno essai pour le dosage de la $\beta2$ microglobuline humaine dans des sérums normaux, d'insuffisants rénaux et dans des liquides de dialyse.

### TABLEAU I :

### Concentration en $\beta2$ micro-globuline

### en µg/mL

| | | | | | | | | |
|---|---|---|---|---|---|---|---|---|
| Procédé selon l'invention | 11,6 | 5,7 | 0,7 | 2,97 | 2,24 | 2,13 | 2,71 | 2,23 |
| Radio-immuno essai | 13,2 | 6,7 | 0,41 | 2,06 | 2,12 | 2,05 | 2,60 | 2,30 |

De la présente invention, outre les avantages précédemment soulignés, se dégagent d'autres avantages parmi lesquels on peut citer :

- le dosage possible en situ, c'est-à-dire au lit même du malade, chose qui était rendue très difficile jusqu'à présent du fait de l'importance de l'appareillage à mettre en oeuvre,
- une très grande rapidité de mise en oeuvre et de détermination des résultats et ce avec une très grande fiabilité, ce qui rend possible ce procédé en biochimie ou en pharmacologie d'urgence et dans le suivi des greffes,
- l'anayse possible dans toute la zone des températures ambiantes habituelles.

Cette invention est donc particulièrement adaptée à de nombreux domaines de recherches et de la médecine, en particulier en cancérologie, hormonologie, endocrinologie, biochimie clinique, toxicologie et microbiologie.

D'autre part, elle présente un grand intérêt dans le domaine du contrôle pyrétogène de l'industrie pharmaceutique et dans celui de l'hémodialyse.

## Revendications

1. Procédé de dosage d'une substance d'intérêt clinique réactive immunologiquement par d'autres substances du même type (dosage d'un antigène par un anticorps et réciproquement), du type selon lequel :
   - on greffe préalablement une substance active immunologiquement, jouant le rôle de réactif doseur sur des microparticules naturelles ou synthétiques,
   - on agglutine lesdites microparticules sur la substance à doser d'intérêt clinique réactive immunologiquement,
   - on mesure, par lecture, le résultat issu de la réaction d'agglutination que l'on compare à une gamme étalon, caractérisé en ce que la phase d'agglutination s'effectue en soumettant lesdites microparticules à un mouvement périodique de fréquence comprise entre 4 et 40 Hertz et d'amplitude variant de quelques millimètres à quelques centimètres.

2. Procédé de dosage selon la revendication 1, caractérisé en ce que la trajectoire du mouvement période est circulaire, rectiligne ou ovalisée.

3. Procédé de dosage selon l'une des revendications 1 et 2, caractérisé en ce que la phase d'agglutination s'effectue à température ambiante.

4. Procédé de dosage selon l'une des revendications 1 à 3, caractérisé en ce que la durée du mouvement périodique imposée aux microparticules durant la phase d'agglutination est fonction de la fréquence, de la trajectoire, de l'amplitude du mouvement et de l'ordre de 10 minutes pour une fréquence de 20 Hertz et un mouvement circulaire périodique de 4 millimètres de diamètre.

FIG.1

FIG.2

# FIG.3

# FIG. 4

Office européen
des brevets

**RAPPORT DE RECHERCHE
EUROPEENNE**

Numéro de la demande

**EP 91 10 1049**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | EP-A-0 186 946   (VIRAL ANTIGENS INC.)<br>* Abrégé; page 8, ligne 29 - page 9, ligne 8; page 12, ligne 20 - page 13, ligne 11 *<br>– – – | 1-4 | G 01 N<br>33/543 |
| D,A | EP-A-0 005 978   (WARNER-LAMBERT CO.)<br>* Document en entier *<br>– – – | 1-4 | |
| D,A | EP-A-0 189 389   (INSTITUTE INTERNATIONAL DE PA-THOLOGIE CELLULAIRE ET MOLECULAIRE)<br>* Page 7, ligne 27 - page 8, ligne 4; page 10, lignes 4-22; revendications 2,3,5-9 *<br>– – – | 1-4 | |
| D,A | US-A-3 488 156   (D.I. GOOD et al.)<br>* L'abrégé *<br>– – – – – | 1-4 | |

**DOMAINES TECHNIQUES
RECHERCHES (Int. Cl.5)**

G 01 N

Le présent rapport de recherche a été établi pour toutes les revendications

| Lieu de la recherche | Date d'achèvement de la recherche | Examinateur |
|---|---|---|
| La Haye | 12 mars 91 | GRIFFITH G. |

CATEGORIE DES DOCUMENTS CITES
X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons
 

& : membre de la même famille, document correspondant